# EUROPEAN PATENT APPLICATION

(11) **EP 4 781 933 A1**
(43) Date of publication of application: **29.07.2026**
(21) Application number: 26151785.8
(22) Date of filing: 14.01.2026
(51) Int. Cl.: A61B 18/12, A61M 1/00

(54) **PUMP CONNECTOR ADAPTER FOR SUCTION OUTLET OF ELECTROSURGICAL DEVICE**

(30) Priority: 28.01.2025 GB 202501177
(71) Applicant: Primamed Limited, Cheltenham, Gloucestershire, GL51 6TQ (GB)
(72) Inventor: Brooke, Gerard Michael, Cheltenham, GL51 6TQ (GB)
(74) Representative: Doherty, William

(57) **Abstract**

A pump connector adapter (110) for an electrosurgical device is provided. The adapter (110) has a body (112) having a flow passage therethrough. An absorption member (130) can then be provided to collect fluid within the body (112).

## Description

The present invention relates to a pump connector adapter for a suction outlet of an electrosurgical device. The invention further relates to an electrosurgical device having such a pump connector adapter and to a vacuum pump apparatus having such a pump connector adapter.

In the field of electrosurgery, it is common to provide radio frequency current to an electrosurgical implement in order to enable surgical actions, in the form of tissue dissection and coagulation, to be performed on a patient. During the process, vapourised cellular matter will be generated as a result of tissue ablation, which must be extracted in order to avoid causing damage to the lungs of the surgeon and attending surgical staff.

This is commonly achieved by the integration of a suction port into, or adjacent to, the distal end of the body of the electrosurgical implement being used. The device, or adjacent suction device, will be connected to a vacuum pump via hosing and an adapter, thereby providing the vacuum effect necessary to extract the effluent.

A common problem with placing suction so close to the surgical site is that naturally, in addition to capturing surgical smoke, bodily fluids can often be aspirated into the electrosurgical implement and suction tubing. This causes several issues. Firstly, contact with fluid severely compromises the integrity of the primary ultra-low particulate air (ULPA) filter contained within the pump unit, intended to remove the harmful compounds present in surgical smoke, thereby rendering it ineffective as a protection mechanism. Secondly, the ingress of bodily fluids into a medical device intended for multiple uses evidently poses a serious contamination hazard, especially if the patient is a carrier of blood-based diseases (for, example, hepatitis). Furthermore, vacuum pumps themselves can be quite sensitive to fluid ingress, resulting in potential failure of the vacuum pump itself if fluid enters into components which are not resilient to such.

Traditionally in the art, to attempt to prevent the ingress of fluid, single use fluid container units have been utilised, positioned between the tubing adaptor of the electrosurgical implement and the suction inlet of the pump unit. However, this is an exceedingly costly and inefficient mechanism for protecting the vacuum unit as, not only must the container be disposed of after each procedure but, its positioning between the implement and the vacuum unit causes a measurable deterioration in the performance of the pump and, thus, the efficacy of the system in respect of the removal of surgical smoke.

The object of the present invention is to provide an improved means of preventing fluid ingress, filter damage and cross-contamination of vacuum pumps used within electrosurgical applications.

According to a first aspect of the invention, there is provided a pump connector adapter for a suction outlet of an electrosurgical device, the pump connector adapter comprising: a body having a first end having a flow inlet configured to connect tubing for the electric surgical device and a second end having a flow outlet configured to connect to a vacuum pump, the body being formed as a unitary member; a flow passage being formed through the body; and an absorption member positioned between the first and second ends to prevent fluid ingress into a connected vacuum pump; wherein the absorption member is seatable in the body through the second end.

It is beneficial to provide a means of inhibiting fluid ingress into vacuum pumps in a manner which allows for easy replacement of a filter or absorption member.

A unitary body allows for very simple removal from a vacuum pump to allow for filter replacement.

Preferably, the absorption member may be a sponge.

A sponge provides a suitable means of absorption of liquids from the fluid flow through the adapter, and is solid enough to be removed from the body without disintegration.

Alternatively, the absorption member may be a paper element.

A paper member can provide the necessary barrier to fluid ingress and can be readily disposed of, without needing to provide a circuitous flow path to accumulate fluid in the adapter.

In one embodiment, the absorption member may have a central receiver aperture engageable around the flow passage.

Given that, in a preferred embodiment, the flow passage is positioned on a central longitudinal axis of the electrosurgical device, the ability to slot the absorption member over the flow passage allows it to be easily removed and exchanged when the absorption member becomes saturated or otherwise needing replacement.

Optionally, the body may be formed as a two-part body.

Having a two-part body allows the pump connector adapter to be opened in order to extract the collected liquids and/or the absorption member. A two-part body is intended to include a body having two separable components, or two parts which open relative to one another, but which may be interconnected, for instance, via a living hinge.

Optionally, the two-part body may comprise a releasable locking means for engaging the first and second body parts of the body.

To avoid leaking or spilling of liquids from the pump connector adapter, it is desirable to inhibit dislocation of the first and second body parts. A releasable locking means thus allows for easy access to the internals of the body, without compromising fluid-tightness.

Preferably, the flow passage may be provided as a dedicated tube within the body, most preferably coaxially positioned with the flow inlet.

If the flow passage and flow inlet are coaxial with one another, this means that where a baffle is provided, the shaping of the baffle entirely defines the non-linearity of the flow path through the body. Since the baffle is preferably a removable component, this improves the ease with which a circuitous flow path can be assembled.

Preferably, the flow passage may define a fluid capture region within the body which is upstream of an entry of the flow outlet.

A dedicated fluid capture region within the body provides a location into which liquid can be collected which is off the main fluid flow path, so that said liquid does not enter into the flow passage.

The absorption member may be receivable within the fluid capture region.

It is desirable for the absorption member to be physically located within the fluid capture region so that the absorption member is best positioned for absorbing liquids on the flow path.

Optionally, the flow outlet may have a greater width or radius than the flow inlet.

For improved suction, the flow inlet which engages with the electrosurgical device may be smaller than the flow outlet that is associated with the pump. This creates a venturi acceleration within the adapter.

In one embodiment, the flow outlet may have a flared rim, which may facilitate engagement with a vacuum pump.

To ease the engagement between the flow outlet and the vacuum pump, a flared rim may be provided which eases the push fit engagement therebetween. That may obvious the need for a separate fastener. A flared rim may also improve ease of insertion of a filter member into the adapter.

In an alternative embodiment, the baffle may be positioned away from a longitudinal axis of the pump connector adapter, and the flow passage is also positioned away from the longitudinal axis of the pump connector adapter.

Whilst providing all of the components on the central longitudinal axis may simplify alignment, it will be appreciated that an off-axis baffle and flow passage arrangement is equally viable, and may have advantages for collection and extraction of liquids under gravity.

In another alternative embodiment, the baffle may be integrally formed with the body.

Whilst it may be simpler from a manufacturing perspective to form the baffle separately of the body, the co-forming of the two may make alignment of the baffle relative to the flow inlet more straightforward. Additive manufacturing processes may make this more achievable.

In one embodiment, the pump connector adapter may further comprise a baffle associated with the flow passage at or adjacent to the first end to define a non-linear flow path through the body.

The provision of a pump connector adapter which has a non-linear flow path therethrough allows for fluid passage through the adapter whilst providing a region in the body which is positioned so that the forward momentum of suspended liquids within the fluid will be captured in said region instead of following the non-linear flow path. This enables the extraction of fluid, such as bodily fluids, which might otherwise contaminate vacuum pump in an environment where it is desirable to be sterile. The user of such an adapter can advantageously swap out the adapter and or a filter from the adapter in lieu of altering the vacuum pump itself.

Optionally, the baffle may have a width or radius greater than a width or radius of the flow inlet.

A wide baffle will force the fluid flow to the radial extremities of the body, urging the fluid away from any centrally-positioned flow passage through which, for example, smoke extraction may occur.

Preferably, the flow passage may be formed as a tube downstream of the baffle, the flow passage having a width or radius less than a width or radius of the baffle.

It is desirable that the baffle be dimensioned to cover a total width or radius of the flow passage so that there is no direct passage through the body from the flow inlet that would otherwise obviate the non-linear flow path.

Optionally, the baffle may be formed as a removable unit receivable within the body.

For ease of assembly and/or manufacture of the adapter, it is possible to provide the baffle as a dedicated component that can be inserted and removed from the body.

The baffle may comprise a plurality of stabilising ribs engageable with the body.

It is desirable that the baffle remain positioned correctly in place with respect to the flow passage, in order to maintain the non-linear flow path. Stabilising ribs on the edge of the baffle allow for this to be achieved regardless of the orientation of the pump connector adapter.

A first body part of the body may comprise the first end and a housing for receiving the baffle, and a second body part may comprise the second end and the flow passage.

A sensible construction of the body has the baffle part on one side and the flow passage on the other, so that the non-linear flow path is defined between the two parts of the body.

Optionally, the baffle may have an internal receiving volume, an end of the flow passage being receivable within the internal receiving volume, and the battle overlapping the end of the flow passage in an axial direction.

The overlapping of the baffle and flow passage in the axial direction makes for a return on the flow path. This makes it extremely difficult for suspended liquid in the evacuated gas to escape through the flow passage without first contacting and thus being absorbed by the absorption member.

The baffle and flow passage may together define a circuitous flow path from the flow inlet to the flow outlet.

A circuitous flow path increases the difficulty of escape of liquid from the pump connector adapter since liquid with forward momentum cannot travel in the opposite direction without first entering the region with the absorption member.

According to a second aspect of the invention, there is provided an electrosurgical device comprising: an electrosurgical implement; a suction tube having a suction inlet at an operable end of the electrosurgical device at or adjacent to the electrosurgical implement and a suction outlet; and a pump connector in accordance with the first aspect of the invention.

It is desirable to provide an electrosurgical device having the capability to extract fluids sucked into the suction inlet before entering into the vacuum pump apparatus.

Optionally, the pump connector adapter may be releasably engageable with the suction outlet.

The ability to completely remove the adapter from the electrosurgical device may improve the ease with which the adapter can be cleaned.

In alternative embodiment, the pump connector adapter may be integrally formed with the suction outlet.

The electrosurgical device having an integral adapter can be provided in a form which is effectively plug-and-play with any appropriate vacuum pump apparatus electrosurgical device.

According to a third aspect of the invention, there is provided a vacuum pump apparatus comprising: a vacuum pump having a pump inlet; and a pump connector adapter in accordance with the first aspect of the invention associated with the pump inlet.

It is desirable to provide a means of extracting fluids ingested by an electrosurgical devices before entering into the vacuum pump apparatus.

Optionally, the pump connector adapter may be releasably engageable with the pump inlet.

The ability to completely remove the adapter from the vacuum pump may improve the ease with which the adapter can be cleaned.

Alternatively, the pump connector adapter may be integrally formed with the pump inlet.

The vacuum pump apparatus having an integral adapter can be provided in a form which is effectively plug-and-play with any appropriate electrosurgical device.

The invention will now be more particularly described, by way of example only, with reference to the accompanying drawings, in which:
Figure 1 shows an exploded isometric representation of a first embodiment of a pump connector adapter in accordance with the first aspect of the invention;
Figure 2 shows a longitudinal cross-sectional representation through the pump connector adapter of Figure 1;
Figure 3 shows a second embodiment of a pump connector adapter in accordance with the first aspect of the invention;
Figure 4 shows an exploded isometric representation of a third embodiment of a pump connector adapter in accordance with the first aspect of the invention; and
Figure 5 shows a longitudinal cross-sectional representation through the pump connector adapter of Figure 4.

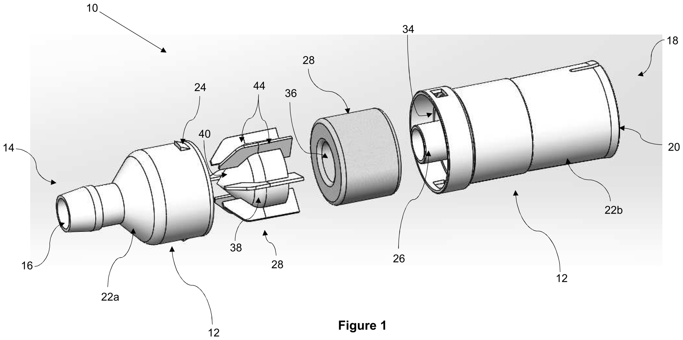

Referring firstly to Figure 1, there is shown a pump connector adapter, indicated globally at 10 and which is suitable for interconnecting a suction tubing of an electrosurgical device with a vacuum pump.

The pump connector adapter 10 comprises a body 12 including a first end 14 having a flow inlet 16 configured to connect to suction tubing for the electrosurgical device and a second end 18 having a flow outlet 20 configured to connect to a vacuum pump. In a usual embodiment, the flow outlet 20 will have a larger width or radius than the flow inlet 16, typically dictated by the comparative sizes of the connection ports on the electrosurgical device and vacuum pump apparatus.

In the depicted embodiment, the body 12 comprises a first body part 22a and a second body part 22b which interconnect to form the assembled body 12. The first and second body parts 22a, 22b may need to connect at an interface 24 having a locking means, such as the depicted press latch.

Fluid flow through the pump connector adapter 10 progresses from the flow inlet 16 to the flow outlet 20 via a flow passage 26 internal to the body 12. Here, the flow passage 26 is formed as a cylindrical tube extending along a longitudinal central axis of the body 12.

Also positioned within the body 12, and preferably but not necessarily as a removable component, is a baffle 28 located between the flow inlet 16 and the flow passage 26 so as to define a non-linear flow path through the body 12. To achieve the non-linear flow path, the baffle 28 preferably has a width or radius greater than that of the flow passage 26.

The purpose of the non-linear flow path is to extract any suspended fluids from the gas being sucked through the pump connector adapter 10. The forward momentum of the suspended fluid will cause said fluid to hit an absorption member 30 located within the body 12 so as to be downstream of the baffle 28 and thereby collect fluid within the body 12.

The first body part 22a is dimensioned so as to accommodate the baffle 28 but the flow inlet 16 has a width or radius designed to be inserted into a suction tube of the electrosurgical device to be used. The end of the flow inlet 16 may be tapered so as to permit an interference fit with the internal bore of the suction tube. The width or radius of the baffle 28 is preferably greater than that of the flow inlet 16 to prevent ejection of the baffle 28.

On the other hand, the second body part 22b may be dimensioned to have a substantially uniform width or radius which is sized to match and/or meet with a connector of a vacuum pump. To assist with connection to a vacuum pump, an end or rim 32 of the second body part 22b maybe or substantially be flared, again allowing for an interference fit with a corresponding connector.

The positioning of the flow passage 26 as a tubular member within the second body part 22b and on the central longitudinal axis create a fluid capture region 34 within the body 12, and which here has an annular or substantially annular volume.

The absorption member 30 is dimensioned to match the shape of the fluid capture region 34; as such, the absorption member 30 has a tubular or annular shape, with a central receiver aperture 36, so as to be readily received within the fluid capture region 34 for capturing fluid entering therein. The absorption member 30 is formed from a sponge material for a similar absorptive material such as gauze or cotton.

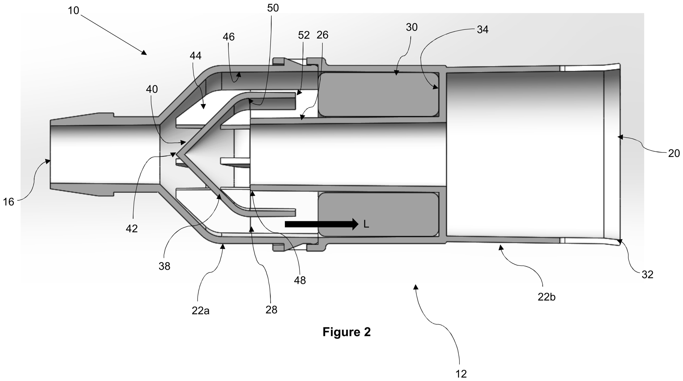

The baffle 28 is sized so as to be received within the first body part 22a, and here has a main baffle element 38 having a flow-directing surface 40 which is directed towards the flow inlet 16. The baffle 28 in the depicted embodiment has a pointed end 42 to form the flow-directing surface 40. In addition, the baffle 28 has a plurality of stabilising ribs, here formed as radially extending vanes 44, which abut an internal surface of the first body part 22a to retain the baffle 28 in place. The radially extending vanes 44 are spaced apart from one another in a circumferential direction of the baffle 28 to thereby leave flow passages around the perimeter of the baffle 28 so that fluid can flow from the flow inlet 16 around the baffle 28. The internal configuration of the pump connector adapter 10 is shown in more detail in Figure 2.

The baffle 28 is received within the first body part 22a, preferably entirely so. The radially extending vanes 44 retain the baffle in place so that the perimetric flow passages have a uniform size and spacing away from a wall 46 of the body 12.

The flow passage 26 extends from the second body part 22b when assembled. A forward end 48 of the flow passage 26 is received within an internal receiving volume 50 of the baffle 28, defined by the main baffle element 38. The main baffle element 38 thus overlaps the forward end 48 of the flow passage 26. A serpentine or circuitous flow path is thus formed between the baffle 28 and the flow passage 26. The flow passage 26 is formed coaxially with the flow inlet 16 to allow receipt into the internal receiving volume 50. The radially extending vanes 44 may extend into the internal receiving volume 50 to form a stop or seat for the forward end 48 of the flow passage 26.

The flow-directing surface 40 is arranged to direct fluid flow through the body 12 at an inner perimeter of the body 12, that is, at or adjacent the wall 46 of the body 12. Around the flow passage 26, within the second body part 22b, and on a linear path L extending from the flow-directing surface 40 is the fluid capture region 34. Fluid flow should thus contact the absorption member 30 in the fluid capture region 34 in preference to turning the first corner of the circuitous flow path defined by the rim 52 of the main baffle element 38. This encourages liquid absorption by the absorption member 30, whilst still allowing for gas suction through the flow passage 26.

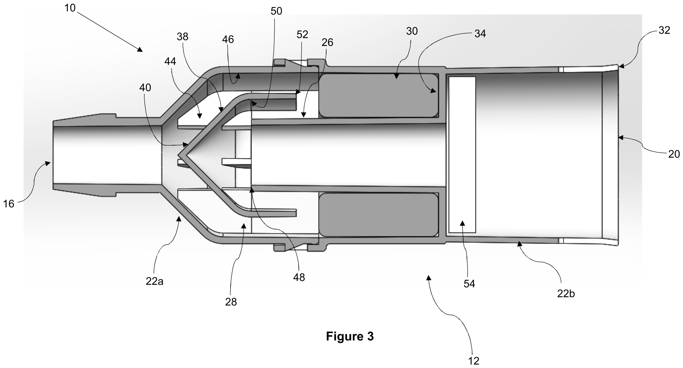

A further embodiment of the pump connector adapter 10 is shown in Figure 3. The pump connector adapter 10 is identical to that of the first embodiment, save for the presence of a filter member 54 located in the second body part 22b downstream of the flow passage 26. This may be loosely held within the flow outlet 20, or there may be a more well-defined locator or holder within the second body part 22b. The filter member 54 may preferably be a HEPA filter. Whilst the baffle has been hereto described as a centrally positioned component allowing flow around the outside thereof, it will be apparent that an equally feasible arrangement could be constructed with a perimetric baffle and a perimetric flow passage, that is where the fluid capture region is coaxial with the flow inlet.

In a further possible alternative embodiment, the baffle may be asymmetric within the pump connector adapter, and still capable of providing a fluid flow path through the body, in conjunction with the flow passage, which is non-linear.

The baffle could additionally be provided as an integral component of the body, rather than as a removable component as hereto described. This may be readily achievable using additive manufacturing techniques.

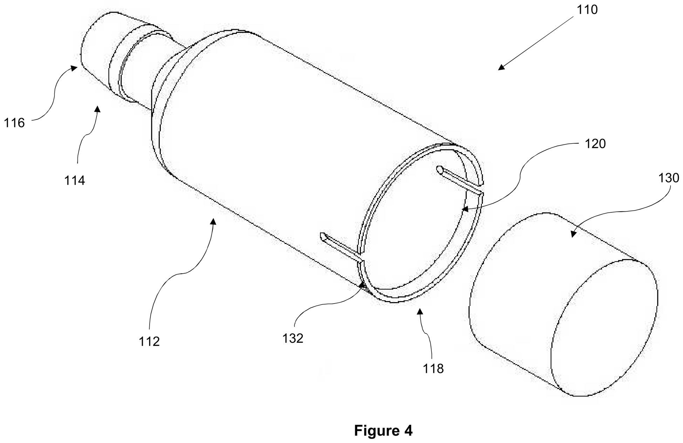

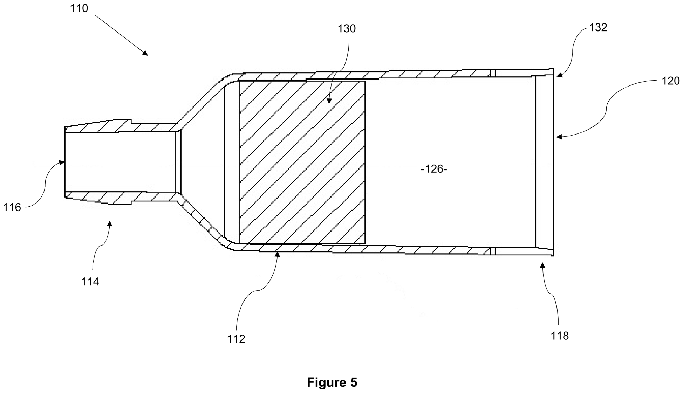

A different embodiment of a pump connector adapter is shown in Figures 4 and 5, referenced globally at 110. Identical or similar features to the first two embodiments will be referenced using identical or similar reference numerals, and further detailed description is omitted for brevity.

The pump connector adapter 110 comprises a body 112 including a first end 114 having a flow inlet 116 configured to connect to suction tubing for the electrosurgical device and a second end 118 having a flow outlet 120 configured to connect to a vacuum pump.

In this embodiment, the body 112 is formed as a unitary component. As such, items, such as filters, can be inserted into the body 112 from the second end 118. The flow passage 126 thus extends linearly through the body 112 without deviation. No baffle is required.

Instead, an absorption or filter member 130 is provided which seats into the body 112 from the second end 118, and which is, in the depicted embodiment formed as a paper cylinder. The flared rim 132 assists with ease of insertion of the filter member 130 into the body 112.

It is therefore possible to provide a pump connector adapter which is suitable for use with or as part of an electrosurgical device and/or with or as part of a vacuum pump apparatus. The pump connector adapter has an integral fluid trap or filter which prevents ingress of fluid into the vacuum pump apparatus, maintaining a sterile environment for longer.

The words 'comprises/comprising' and the words 'having/including' when used herein with reference to the present invention are used to specify the presence of stated features, integers, steps, or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein.

## Claims

1. A pump connector adapter (10; 110) for a suction outlet of an electrosurgical device, the pump connector adapter comprising:
a body (12; 112) including a first end (14; 114) having a flow inlet (16; 116) configured to connect to tubing for the electrosurgical device and a second end (18; 118) having a flow outlet (20; 120) configured to connect to a vacuum pump, the body (12; 112) being formed as a unitary member;
a flow passage (26; 126) being formed through the body (12; 112); and
an absorption member (30; 130) positioned between the first and second ends (14, 18; 114, 118) to prevent fluid ingress into a connected vacuum pump;
wherein the absorption member (30; 130) is seatable in the body (12; 112) through the second end (18; 118).

2. A pump connector adapter (10; 110) as claimed in claim 1, wherein the absorption member (30; 130) is a sponge or a paper element.

3. A pump connector adapter (10; 110) as claimed in claim 1 or claim 2, wherein the absorption member (30) has a central receiver aperture (36) engageable around the flow passage (26; 126).

4. A pump connector adapter (10; 110) as claimed in any one of claims 1 to 3, wherein the flow passage (26; 126) is provided as a dedicated tube within the body.

5. A pump connector adapter (10) as claimed in any one of the preceding claims, wherein the flow passage (26) defines a fluid capture region (34) within the body (12) which is upstream of an entry of the flow outlet (20).

6. A pump connector adapter (10) as claimed in claim 5, wherein the absorption member (30) is receivable within the fluid capture region (34).

7. A pump connector adapter (10; 110) as claimed in any one of the preceding claims, wherein the flow outlet (20; 120) has a greater width or radius than the flow inlet (16; 116).

8. A pump connector adapter (10; 110) as claimed in any one of the preceding claims, wherein the flow outlet (20; 120) has a flared rim.

9. A pump connector adapter (10) as claimed in any one of the preceding claims, further comprising a baffle (28) associated with the flow passage (26) at or adjacent to the first end (14) to define a non-linear flow path through the body (12).

10. A pump connector adapter (10) as claimed in claim 9, wherein the baffle (28) has a width or radius greater than a width or radius of the flow inlet (16).

11. A pump connector adapter (10) as claimed in claim 9 or claim 10, wherein the flow passage (26) is formed as a tube downstream of the baffle (28), the flow passage (26) having a width or radius less than a width or radius of the baffle (28).

12. A pump connector adapter (10) as claimed in any one of claims 9 to 11, wherein the baffle (28) is formed as a removable unit receivable within the body (12).

13. A pump connector adapter (10) as claimed in any one of claims 9 to 12, wherein the baffle (28) is integrally formed with the body (12).

14. An electrosurgical device comprising:
an electrosurgical implement;
a suction tube having a suction inlet at an operable end of the electrosurgical device at or adjacent to the electrosurgical implement and a suction outlet; and
a pump connector adapter (10; 110) as claimed in any one of the preceding claims.

15. A vacuum pump apparatus comprising:
a vacuum pump having a pump inlet; and
a pump connector adapter (10; 110) as claimed in any one of claims 1 to 13 associated with the pump inlet.
